# EUROPÄISCHE PATENTSCHRIFT

(11) **EP 3 796 963 B1**
(45) Veröffentlichungstag und Bekanntmachung des Hinweises auf die Patenterteilung: **17.08.2022**
(21) Anmeldenummer: 19714306.8
(22) Anmeldetag: 19.03.2019
(51) Int. Cl.: A24F 40/44, A24F 40/70, A61M 15/06, A61M 11/04

(54) **VERDAMPFERKÖRPER FÜR EINE VERDAMPFERVORRICHTUNG EINES INHALATORS**
EVAPORATOR UNIT FOR AN EVAPORATOR DEVICE OF AN INHALER
CORPS D'ÉVAPORATEUR POUR UN DISPOSITIF ÉVAPORATEUR D'UN INHALATEUR

(30) Priorität: 23.05.2018 AT 504182018
(43) Veröffentlichungstag der Anmeldung: 31.03.2021
(73) Patentinhaber: Von Erl. GmbH, 6060 Hall In Tirol (AT); MCI Management Center Innsbruck - Internationale Hochschule GmbH, 6020 Innsbruck (AT)
(72) Erfinder: NEUNER, Reinhard, 6105 Leutasch (AT); STÄRZ, Ronald, 6073 Sistrans (AT); HUPFAUF, Benjamin, 6521 Fließ (AT)
(74) Vertreter: Gulde & Partner
(86) Internationale Anmeldenummer: PCT/AT2019/060091
(87) Internationale Veröffentlichungsnummer: WO 2019/222774

(56) Entgegenhaltungen:
- WO-A1-2015/198049
- WO-A1-2016/079151
- WO-A1-2016/096728
- WO-A1-2017/207416
- WO-A1-2018/037206
- WO-A2-2016/150922
- WO-A2-2018/078546
- US-A1- 2016 338 402
- US-A1- 2017 049 153
- US-A1- 2017 340 014
- US-A1- 2018 020 737
- US-A1- 2018 132 534

## Beschreibung

Die vorliegende Erfindung betrifft einen Verdampferkörper mit den Merkmalen des Oberbegriffs des Anspruchs 1, eine Verdampfervorrichtung mit einem solchen Verdampferkörper, einen Inhalator und ein Mundstück für einen Inhalator mit einer Verdampfervorrichtung sowie Verfahren zur Herstellung eines Verdampferkörpers.

Inhalatoren wie beispielsweise Elektrozigaretten, die häufig auch als elektrische oder elektronische Zigaretten bezeichnet werden, enthalten üblicherweise eine Verdampfervorrichtung zum Verdampfen einer flüssigen Substanz, auch Liquid genannt. Typische Verdampfervorrichtungen umfassen einen Verdampferkörper (manchmal auch als Verdampferkern bezeichnet), dem das Liquid zuführbar ist, sowie eine Heizvorrichtung, die im oder am Verdampferkörper angeordnet ist und die das im Verdampferkörper vorhandene Liquid erhitzt, bis es verdampft. Beim Saugen an einem Mundstück des Inhalators entsteht im Inneren des Inhalators üblicherweise ein Luftstrom, der sich mit dem verdampften Liquid anreichert und in weiterer Folge wird das Gemisch aus Luft und verdampftem Liquid durch den Benutzer des Inhalators inhaliert.

Bekannte Verdampferkörper umfassen Baumwolle, die mit einem Liquid, das üblicherweise in einem Tank innerhalb des Inhalators bereitgestellt wird, in Verbindung steht. Um den Verdampferkörper ist häufig eine Heizvorrichtung in Form einer Heizwendel gewickelt. Der aus Baumwolle bestehende Verdampferkörper saugt das Liquid auf und die Heizwendel erhitzt das Liquid, bis es verdampft. Baumwolle ist ein Naturprodukt, dessen Eigenschaften teilweise großen Schwankungen unterworfen sind. So besteht Baumwolle aus einzelnen Baumwollfasern, deren Faserlängen, Faserdurchmesser und Zugfestigkeiten große Schwankungen aufweisen können. Dementsprechend ist bei gegebener Form des Verdampferkörpers auch die Aufnahmekapazität von Liquid, also wie viel Liquid vom Verdampferkörper aufgenommen werden kann, je nach verwendeter Baumwolle unterschiedlich hoch. In Bezug auf die Baumwolle für Verdampferkörper ist es zudem so, dass es auf dem Markt nur ganz wenige Anbieter gibt und man somit völlig von der Qualität der von diesen Anbietern angebotenen Baumwolle abhängig ist. Darüber hinaus ist die Fertigung herkömmlicher Verdampferkörper überwiegend manuell, wodurch Verdampferkörper mit unterschiedlichen Dichten produziert werden. Auch der Zusammenbau des Verdampferkörpers mit einer Heizwendel geschieht meist manuell, sodass die Heizwendeln die Verdampferkörper unterschiedlich eng umwickeln. Aus all dem resultiert ein nicht vorhersagbares Verdampferverhalten, das von der konkreten Zusammensetzung der Baumwolle und der jeweiligen manuellen Fertigung des Verdampferkörpers abhängt. Das Verdampferverhalten bzw. das Dampfergebnis herkömmlicher Verdampferkörper ist kaum vorhersagbar und vor allem zwischen verschiedenen, in einer Serie gefertigten, Verdampferkörpern nicht gleichbleibend sondern stark schwankend.

In der US 2017/049153 A1 wird ferner eine Verdampfervorrichtung mit einem Flüssigkeitsabsorber und einem Heizelement offenbart, welche in Kontakt miteinander gebracht sind, und wobei der Flüssigkeitsabsorber aus verschiedenen darin offenbarten Materialien bestehen kann. In der WO 2016/150922A2 und in der WO 2016/150922 A2 werden weitere Verdampfervorrichtungen gezeigt mit Absorbern verschiedener Materialen. In den Dokumenten WO 2016/079151 A1, WO2018/078546 A2, US 2018/078546 A2, WO2015/198049 A1, WO 2018/037206 A1, WO 2017/207416 A1, US 2018/020737 A1 und US 2016/338402 A1 werden weitere Verdampfervorrichtungen mit Verdampferkörpern des Standes der Technik beschrieben.

Aufgabe der Erfindung ist es, einen gegenüber dem Stand der Technik verbesserten Verdampferkörper, eine Verdampfervorrichtung mit einem solchen Verdampferkörper sowie einen Inhalator und ein Mundstück für einen Inhalator mit einer derart verbesserten Verdampfervorrichtung bereitzustellen. Insbesondere soll ein Verdampferkörper mit einem besser vorhersagbaren Verdampferverhalten bereitgestellt werden. Außerdem soll ein verbessertes Verfahren zur Herstellung eines Verdampferkörpers angegeben werden.

Diese Aufgabe wird durch einen Verdampferkörper mit den Merkmalen des Anspruchs 1, eine Verdampfervorrichtung mit einem solchen Verdampferkörper, einen Inhalator und ein Mundstück für einen Inhalator mit einer solchen Verdampfervorrichtung sowie durch Verfahren zur Herstellung solcher Verdampferkörper.

Vorteilhafte Ausführungsformen der Erfindung sind in den abhängigen Ansprüchen definiert.

Bei der Erfindung ist vorgesehen, dass der Verdampferkörper aus einem Material besteht, das wenigstens einen thermoplastischen Kunststoff, vorzugsweise ein Hochleistungsthermoplast, umfasst, der mit dem wenigstens einen thermoplastischen Kunststoff zur Bereitstellung einer Mischung vermengt ist.

Durch die Herstellung des Verdampferkörpers aus einem Material umfassend wenigstens einen thermoplastischen Kunststoff, bei dem es sich vorzugsweise um ein Hochleistungsthermoplast handelt, ist es möglich, eine Mehrzahl von Verdampferkörpern herzustellen, die alle dieselben Materialeigenschaften besitzen. Dadurch ist das Verdampferverhalten der Verdampferkörper besser vorhersagbar und nicht mehr so großen Schwankungen unterworfen.

Ein Hochleistungsthermoplast ist ein thermoplastischer Kunststoff bzw. Thermoplast, welcher Gebrauchstemperaturen im Sinne der Anwendung aufweist und die Verarbeitung im Sinne der Erfindung zulässt, sowie die notwendigen chemischen und mechanischen Eigenschaften besitzt, welche sich im Sinne der Anwendung und Verarbeitung ergeben. Die Gebrauchstemperatur umfasst demnach jenen Temperaturbereich, der für die Verdampfung des Liquids zweckmäßig ist. Ferner sollte ein solcher Thermoplast einer der Anwendung und Verarbeitung entsprechenden zeitlichen Beanspruchung standhalten.

Im Allgemeinen wird zwischen teilkristallinen und amorphen Thermoplasten unterschieden, wobei für teilkristalline Thermoplaste üblicherweise ein Schmelzpunkt und für amorphe Thermoplaste eine Glasübergangstemperatur angegeben wird. Sobald die Glasübergangstemperatur überschritten ist, kommt es zu einer Erweichung.

Da ein Bestandteil gängiger Liquids für Elektrozigaretten Propylenglycol mit einer Verdampfungstemperatur von ca. 188 °C ist, sollte der verwendete Thermoplast (z.B. ein Polymer) eine entsprechend höhere Schmelztemperatur bzw. eine Glasübergangstemperatur von ca. > 88 °C aufweisen. Da der Verdampferkörper einer Elektrozigarette keiner mechanischen Beanspruchung standhalten muss, kann eine Erweichung durchaus erlaubt sein, wodurch sich eine gegenüber der Verdampfungstemperatur niedrigere Anforderung hinsichtlich der Glasübergangstemperatur für den Thermoplast ergibt. Eine weitere Anwendung verwendet ein Liquid umfassend Glycerin, welches bei ca. 290 °C verdampft. Der entsprechende Thermoplast würde deshalb vorzugsweise eine Schmelztemperatur von > 290 °C bzw. eine Glasübergangstemperatur von ca. > 190 °C aufweisen. In einem besonders bevorzugten Fall wird eine Liquid-Mischung verwendet, welche bei ca. 250 °C verdampft. Ein entsprechender Thermoplast beispielsweise in Form eines teilkristallinen Polymers würde vorzugsweise eine Schmelztemperatur > 250 °C aufweisen und ein entsprechender Thermoplast beispielsweise in Form eines amorphen Polymers würde vorzugsweise eine Glasübergangstemperatur von ca. > 150 °C aufweisen.

Vorzugsweise kann daher vorgesehen sein, dass eine Schmelztemperatur des wenigstens einen thermoplastischen Kunststoffs größer etwa 180 °C, vorzugweise größer etwa 250 °C, beträgt.

Ebenso kann vorzugsweise vorgesehen sein, dass eine Glasübergangstemperatur des wenigstens einen thermoplastischen Kunststoffs größer etwa 80 °C, vorzugweise größer etwa 150 °C, beträgt.

Beispielsweise kann ein Thermoplast, welcher den oben genannte Ansprüchen genügt, ein Poly-Aryl-Ether-Keton bzw. Polyaryletherketon (PAEK), vorzugsweise Poly-Ether-Ether-Keton bzw. Polyetheretherketon (PEEK), sein.

Weitere geeignete Thermoplaste sind Poly-Sulfone wie Polysulfon (PSU), Poly-Ether-Sulfon (PES), Poly-Phenyl-Sulfon (PPSU) oder Polyimide (PI) wie Poly-Ether-Imid (PEI), Poly-Amid-Imid (PAI) oder Poly-Phenylen-Sulfid (PPS), Polyamid (PA), Poly-Phenylen-Ether (PPE), Poly-Phtal-Amid (PPA). Darüber hinaus können CoPolymerisate oder Mischungen daraus, sowie gefüllte, ungefüllte oder faserverstärkte Thermoplaste verwendet werden.

Vorzugsweise kann daher vorgesehen sein, dass das Material des Verdampferkörpers wenigstens einen der folgenden thermoplastischen Kunststoffe umfasst: ein Poly-Aryl-Ether-Keton (PAEK), vorzugsweise Poly-Ether-Ether-Keton (PEEK); ein Poly-Sulfon, vorzugsweise Polysulfon (PSU) und/oder Poly-Ether-Sulfon (PES) und/oder Poly-Phenyl-Sulfon (PPSU); ein Polyimid (PI), vorzugsweise Poly-Ether-Imid (PEI) und/oder Poly-Amid-Imid (PAI); Poly-Phenylen-Sulfid (PPS); Polyamid (PA); Poly-Phenylen-Ether (PPE); Poly-Phtal-Amid (PPA).

Besonders bevorzugt kann vorgesehen sein, dass der Verdampferkörper im Wesentlichen vollständig aus wenigstens einem Polyaryletherketon, vorzugsweise Polyetheretherketon, besteht. Es ist aber auch möglich, dass das Material des Verdampferkörpers neben einem Polyaryletherketon noch Additive und/oder elektrisch leitfähige Zusatzstoffe enthält.

Polyaryletherketone, abgekürzt PAEK, sind hochtemperaturbeständige thermoplastische Kunststoffe, auch Hochleistungsthermoplaste genannt. Sie verfügen über eine herausragende mechanische Stabilität sowie über chemische Resistenz gegen eine Vielzahl an Lösungsmitteln. Ein weit verbreiteter Vertreter dieser hochtemperaturfesten Werkstoffe ist das Polyetheretherketon, abgekürzt PEEK. PEEK schmilzt erst bei einer verhältnismäßig hohen Temperatur von 335 °C und kann im Spritzgussverfahren oder per Extruder geformt werden. Im festen Zustand kann PEEK einfach weiterverarbeitet (zum Beispiel geschnitten oder gefräst) werden. Der Einsatz von Polyaryletherketonen als Material für den Verdampferkörper ermöglicht es, eine Mehrzahl von Verdampferkörpern herzustellen, die alle dieselben Materialeigenschaften besitzen. Dadurch ist das Verdampferverhalten der Verdampferkörper besser vorhersagbar und nicht mehr so großen Schwankungen unterworfen.

Gemäß einer besonders bevorzugten Ausführungsform kann vorgesehen sein, dass der Verdampferkörper porös ausgebildet ist. Eine poröse, also Hohlräume aufweisende, Struktur des Verdampferkörpers erhöht die Aufnahmekapazität des Verdampferkörpers für das dem Verdampferkörper im Gebrauch zuzuführende Liquid.

Vorzugsweise kann dabei vorgesehen sein, dass der Verdampferkörper eine offene Porosität von etwa 30 % bis 96 %, vorzugsweise von etwa 50 % bis 95 %, besonders bevorzugt von etwa 65 % bis 94 %, aufweist. Die offene Porosität berücksichtigt hierbei die untereinander und mit der Umgebung in Verbindung stehenden Hohlräume der Struktur des Verdampferkörpers und stellt somit den prozentualen Anteil dieser Hohlräume am Gesamtvolumen des Verdampferkörpers dar.

Besonders bevorzugt kann vorgesehen sein, dass der Verdampferkörper selbststragend ist. Eine statisch selbsttragende Ausbildung des Verdampferkörpers erleichtert das Bearbeiten (zum Beispiel schneiden oder fräsen) des Verdampferkörpers und dessen Einbau in einer Verdampfervorrichtung.

Vorzugsweise kann vorgesehen sein, dass das Material des Verdampferkörpers wenigstens ein Additiv umfasst.

So kann dem Material des Verdampferkörpers ein Additiv wie zum Beispiel Kieselgur oder Tonerde beigemengt sein. Derartige Feststoffe, die vorzugsweise in sich porös sein können, verbleiben während der Herstellung des Verdampferkörpers in der Struktur des Verdampferkörpers und können teilweise die Verbindung der Thermoplaste des Verdampferkörpers verhindern, was die Porosität des Verdampferkörpers begünstigt.

Weitere Beispiele von Additiven sind: Fluide, vorzugsweise Wasser und/oder Isopropanol; Treibmittel, vorzugsweise NaHCO₃; lösliche Feststoffe, vorzugweise lösliche Salze (z.B. Chloride, Carbonate oder Halogenide) und/oder NaCl, Na₂CO₃, CaCl₂, KCl, MgCl₂.

Das Material des Verdampferkörpers umfasst erfindungsgemäß wenigstens einen elektrisch leitfähigen Zusatzstoff, wobei vorzugsweise der wenigstens eine elektrisch leitfähige Zusatzstoff ein Metall, Graphit, Graphen, Siliciumcarbid oder Aktivkohle ist. Durch das Beimengen eines elektrisch leitfähigen Zusatzstoffes zum Material des Verdampferkörpers ist der Verdampferkörper selbst in sich elektrisch leitfähig, wodurch er selbst einen Heizwiderstand und somit selbst auch eine Heizvorrichtung darstellen kann.

Wenn der wenigstens eine elektrisch leitfähige Zusatzstoff eine gute Wärmeleitfähigkeit besitzt, wie beispielsweise Metalle oder Siliciumcarbid, kann damit die Wärmeleitung in das zu verdampfende Liquid begünstigt werden.

Gemäß einer bevorzugten Ausführungsform kann vorgesehen sein, dass der Verdampferkörper zylindrisch, vorzugsweise kreiszylindrisch, ausgebildet ist.

Vorzugsweise kann vorgesehen sein, dass eine Oberfläche des Verdampferkörpers wenigstens bereichsweise mit wenigstens einer elektrisch leitfähigen Beschichtung versehen ist. Durch das Vorsehen wenigstens einer elektrisch leitfähigen Beschichtung auf einer Oberfläche des Verdampferkörpers kann auf eine gesonderte Heizvorrichtung in Form einer Heizwendel, die um einen Verdampferkörper herumgewickelt wird und eine nicht genau bestimmbare Kontaktierung mit dem Verdampferkörper eingeht, verzichtet werden. Stattdessen weist der Verdampferkörper wenigstens eine Beschichtung auf, die optimal mit dem Verdampferkörper kontaktiert ist und als Heizvorrichtung dienen kann. Auch dadurch ist somit das Verdampferverhalten der Verdampferkörper besser vorhersagbar und nicht mehr so großen Schwankungen unterworfen.

Gemäß einer bevorzugten Ausführungsform kann außerdem vorgesehen sein, dass der Verdampferkörper zumindest bereichsweise, vorzugsweise im Wesentlichen vollständig, als textiles Gebilde, vorzugsweise als flächiges oder räumliches Gewebe, ausgebildet ist, wobei das textile Gebilde wenigstens eine Faser und/oder wenigstens ein Filament umfassend den wenigstens einen thermoplastischen Kunststoff umfasst.

Bei dem textilen Gebilde kann es sich auch um ein Gewirk, Gestrick, Geflecht, Nähgewirk, Vlies, Filz oder beispielsweise um einen textilen Schlauch handeln.

Generell kann das textile Gebilde flächig (beispielsweise wie ein Filter) oder zylindrisch bzw. rund (beispielsweise wie eine Schnur) ausgeführt sein.

Vorzugsweise kann vorgesehen sein, dass die wenigstens eine Faser und/oder das wenigstens eine Filament im Wesentlichen vollständig aus wenigstens einem Polyaryletherketon, vorzugsweise Polyetheretherketon, besteht bzw. bestehen.

Es kann vorgesehen sein, dass die wenigstens eine Faser und/oder das wenigstens eine Filament einen Durchmesser kleiner 1000 µm, vorzugsweise kleiner 500 µm, besonders bevorzugt kleiner 300 µm, aufweist bzw. aufweisen. In einer bevorzugten Ausführungsform kann der Durchmesser der wenigstens einen Faser und/oder des wenigstens einen Filaments kleiner 50 µm, beispielsweise 38 µm, sein.

Es kann auch vorgesehen sein, dass das textile Gebilde aus wenigstens einem Faden und/oder Garn umfassend die wenigstens eine Faser und/oder das wenigstens eine Filament aufgebaut ist.

Vorzugsweise kann vorgesehen sein, dass das textile Gebilde dochtartig ausgebildet ist. In diesem Fall ist das textile Gebilde besonders gut dafür geeignet, ein Liquid für eine Elektrozigarette aufzunehmen und weiterzutransportieren. Das textile Gebilde kann insbesondere durch Anzahl, Durchmesser und Verarbeitungsart der Fasern und/oder Filamente in seiner offenen Porosität verändert werden, um den Anforderungen als Docht (poröse Struktur) zu genügen. Mögliche Verarbeitungsarten sind beispielsweise: Spinnen, Weben, Wirken, Stricken, Flechten, Filzen, Fügen.

Es kann vorgesehen sein, dass das textile Gebilde wenigstens ein Additiv enthält. Mögliche Additive für das Material des Verdampferkörpers wurden bereits angeführt. Das wenigsten seine Additiv kann bereits in der wenigstens einen Faser bzw. im wenigstens einen Filament enthalten sein (z.B. gefülltes PEEK). Es kann aber auch in Form einer Additiv-Faser oder eines Additiv-Filaments mit der wenigstens einen Faser und/oder dem wenigstens einen Filament verarbeitet sein, beispielsweise verwebt. Beispiele für solche Additiv-Fasern oder Additiv-Filamente sind Metalldrähte oder - bänder sowie Graphit-, Glas- oder Basalt-Filamente. Additiv-Fasern oder Additiv-Filamente können dabei auch als Heizvorrichtung dienen. Vorzugsweise kann es sich bei dem textilen Gebilde um ein PEEK-Gewebe handeln, welches wenigstens ein Additiv enthält.

Das textile Gebilde kann beispielsweise als flächiges Gewebe ausgebildet sein. Das flächige Gewebe kann gerollt, gefaltet oder gestapelt werden. Um eine runde Geometrie zu erhalten, können übliche Webverfahren angewandt werden. Der Zuschnitt des textilen Gebildes kann über eine temperierte Klinge erfolgen, wodurch die Enden der Fasern und/oder Filamente verschmelzen und fixiert werden.

Das textile Gebilde kann auch eine Art Mantel um ein Kernmaterial herum bilden. Das Kernmaterial kann wiederum wenigstens einen thermoplastischen Kunststoff, vorzugsweise ein Hochleistungsthermoplast, umfassen. Beispiele für geeignete thermoplastische Kunststoffe und Hochleistungsthermoplast wurden bereits angeführt. Beispielsweise könnte ein textiles Gebilde in Form eines PEEK-Gewebes um ein Kernmaterial herum gewickelt oder geflochten werden. Das Kernmaterial würde so von Temperaturspitzen, wie sie beispielsweise in einem Verdampfer einer Elektrozigarette auftreten können, abgeschirmt.

Schutz wird auch begehrt für eine Verdampfervorrichtung für einen Inhalator gemäß dem korrespondierenden Anspruch, einen Inhalator gemäß dem korrespondierenden Anspruch und ein Mundstück für einen Inhalator gemäß dem korrespondierenden Anspruch. Vorteilhafte Ausführungsformen sind in den jeweils abhängigen Ansprüchen angegeben.

Vorzugsweise kann vorgesehen sein, dass bei einer Verdampfervorrichtung eine Heizvorrichtung vorgesehen ist, wobei die Heizvorrichtung im oder am Verdampferkörper angeordnet ist. Bei der Heizvorrichtung kann es sich um eine elektrisch leitfähige Beschichtung handeln, die auf einer Oberfläche des Verdampferkörpers angeordnet ist. Es kann sich beispielsweise aber auch um einen elektrisch leitfähigen Draht handeln, der in den Verdampferkörper integriert oder um den Verdampferkörper herumgewickelt ist.

Gemäß einer bevorzugten Ausführungsvariante kann vorgesehen sein, dass die Verdampfervorrichtung einen Aufnahmeraum für ein Liquid umfasst, wobei der Verdampferkörper mit dem Liquid in Verbindung steht.

Darüber hinaus wird Schutz begehrt für ein Verfahren zur Herstellung eines Verdampferkörpers mit den Merkmalen des Anspruchs 14. Vorteilhafte Ausführungsformen sind in den davon abhängigen Ansprüchen angegeben.

Beim vorgeschlagenen Verfahren wird eine Mischung umfassend wenigstens einen thermoplastischen Kunststoff, vorzugsweise ein Hochleistungsthermoplast, bereitgestellt, wobei die Mischung erhitzt wird bis die Mischung zumindest oberflächlich geschmolzen ist oder sich eine Schmelze bildet, wobei aus der zumindest oberflächlich geschmolzen Mischung oder der Schmelze wenigstens ein Verdampferkörper geformt wird.

Die Mischung stellt dabei das Material des Verdampferkörpers dar.

Vorzugsweise kann die Mischung wenigstens einen der folgenden thermoplastischen Kunststoffe umfassen: ein Poly-Aryl-Ether-Keton (PAEK), vorzugsweise Poly-Ether-Ether-Keton (PEEK); ein Poly-Sulfon, vorzugsweise Polysulfon (PSU) und/oder Poly-Ether-Sulfon (PES) und/oder Poly-Phenyl-Sulfon (PPSU); ein Polyimid (PI), vorzugsweise Poly-Ether-Imid (PEI) und/oder Poly-Amid-Imid (PAI); Poly-Phenylen-Sulfid (PPS); Polyamid (PA); Poly-Phenylen-Ether (PPE); Poly-Phtal-Amid (PPA).

Erfindungsgemäß ist vorgesehen, dass die Mischung wenigstens einen elektrisch leitfähigen Zusatzstoff - vorzugsweise ein Metall, Graphit, Graphen, Siliciumcarbid oder Aktivkohle - umfasst, der mit dem wenigstens einen thermoplastischen Kunststoff zur Bereitstellung der Mischung vermengt ist.

Die elektrisch leitfähigen Zusatzstoffe können vorzugsweise eine gute Wärmeleitfähigkeit aufweisen, wie zum Beispiel Metalle oder Siliziumcarbid (SiC). Vorzugsweise sind die Partikeldurchmesser der beigemengten elektrisch leitfähigen Zusatzstoffe kleiner etwa 2000 µm, vorzugsweise kleiner etwa 1000 µm, besonders bevorzugt kleiner etwa 500 µm.

Das Beimengen von vorzugsweise pulverförmigen elektrisch leitfähigen Zusatzstoffen zur Mischung führt dazu, dass der fertig hergestellte Verdampferkörper in sich elektrisch leitfähig ist, wodurch er selbst eine Art Heizwiderstand darstellt, der bei Anlegen einer elektrischen Spannung den Verdampferkörper insgesamt erhitzt.

Es kann vorgesehen sein, dass die Mischung wenigstens ein Additiv umfasst, das mit dem wenigstens einen thermoplastischen Kunststoff zur Bereitstellung der Mischung vermengt wird.

Diese Additive können Feststoffe sein, die auch in sich porös sein können. Diese Additive können während des Herstellungsprozesses des Verdampferkörpers die Verbindung des thermoplastischen Kunststoffs teilweise verhindern, was sich auf die Porosität des Verdampferkörpers auswirken kann. Die Additive können in der Struktur des fertig hergestellten Verdampferkörpers verbleiben.

Beispiele von Additiven sind: Kieselgur; Tonerden; Fluide, vorzugsweise Wasser und/oder Isopropanol; Treibmittel, vorzugsweise NaHCO₃; lösliche Feststoffe, vorzugweise lösliche Salze (z.B. Chloride, Carbonate oder Halogenide) und/oder NaCl, Na₂CO₃, CaCl₂, KCl, MgCl₂.

Kieselgur als Additiv hat den Vorteil, dass es in sich porös ist und somit zu einer erhöhten Porosität des Verdampferkörpers beiträgt. Mit geringen Massenanteilen eines solchen Additivs, von beispielsweise 20 %, können schon ausreichende Porositäten erzeugt werden.

Vorzugsweise sind die Partikeldurchmesser der beigemengten Additive kleiner etwa 2000 µm, vorzugsweise kleiner etwa 1000 µm, besonders bevorzugt kleiner etwa 500 µm.

Es kann auch vorgesehen sein, dass der Mischung wenigstens ein Fluid wie zum Beispiel Wasser oder Isopropanol als Additiv beigemengt wird. Ein derartiges Fluid erleichtert das Vermischen der Mischung und die weitere Verarbeitung, da mit Beigabe eines Fluids das Schüttvolumen verringert werden kann und eine teigige Konsistenz der Mischung erhalten werden kann. Wenn das beigemengte Fluid vor dem oberflächlichen Anschmelzen oder Schmelzen der Mischung nicht entfernt wird (zum Beispiel durch Trocknung), so kann es während des Erhitzens der Mischung als Treibmittel wirken und zusätzliche Porositäten bewirken. Als Treibmittel kann z.B. auch NaHCO₃ (im Handel häufig als "Natron" bezeichnet) beigemengt werden.

Vorzugsweise kann vorgesehen sein, dass die Mischung wenigstens einen löslichen Feststoff, vorzugweise ein Salz umfasst, wobei der wenigstens eine lösliche Feststoff mit dem wenigstens einen thermoplastischen Kunststoff zur Bereitstellung der Mischung vermengt wird, wobei nach dem Formen des wenigstens einen Verdampferkörpers der wenigstens eine lösliche Feststoff zumindest teilweise, vorzugsweise im Wesentlichen vollständig, aus dem Verdampferkörper herausgelöst wird.

Dadurch beinhaltet die Struktur des Verdampferkörpers lösliche Feststoff-Partikel (z.B. in Form von Salzpartikeln), welche die Porosität des Verdampferkörpers beeinflussen. Wenn es sich bei dem wenigstens einen löslichen Feststoff um ein lösliches Salz handelt, kann dieses nach der Formgebung mit einem geeigneten Lösungsmittel wie beispielsweise Wasser (H₂O) aus dem Verdampferkörper herausgelöst werden. Dadurch entstehen untereinander und mit der Umgebung in Verbindung stehenden Hohlräume in der Struktur des Verdampferkörpers, welche die offene Porosität des Verdampferkörpers beeinflussen. Bei den löslichen Salzen kann es sich beispielsweise um Chloride, Carbonate oder Halogenide handeln. Vorzugsweise kann der Mischung NaCl, Na₂CO₃, CaCl₂, KCl, MgCl₂ in Form von löslichen Feststoff-Partikeln beigemischt werden.

Vorzugsweise sind die Partikeldurchmesser der beigemengten löslichen Feststoff-Partikel kleiner etwa 2000 µm, vorzugsweise kleiner etwa 1000 µm, besonders bevorzugt kleiner etwa 500 µm.

Gemäß einer bevorzugten Ausführungsform kann vorgesehen sein, dass der wenigstens eine thermoplastische Kunststoff ein pulver- oder flockenförmiges Polyaryletherketon, vorzugsweise Polyetheretherketon, ist. Vorzugsweise kann dabei vorgesehen sein, dass der Massenanteil des pulver- oder flockenförmigen Polyaryletherketons in der Mischung etwa 4 % bis 65 %, vorzugsweise etwa 5 % bis 55 %, besonders bevorzugt etwa 6 % bis 45 % beträgt.

Für die Mischung kann vorzugsweise ein pulverförmiges Polyetheretherketon verwendet werden mit Partikeldurchmessern kleiner etwa 5000 µm. Vorzugsweise beträgt der Partikeldurchmesser weniger als etwa 1000 µm. Im Handel erhältliche pulverförmige Polyetheretherketon weisen häufig PEEK-Partikel mit Partikeldurchmessern auf, die über einen weiten Bereich gestreut sind. Für derartige Pulver gibt es häufig die Angabe eines mittleren Partikeldurchmessers bzw. der mittleren Korngröße. Vorzugsweise wird ein PEEK-Pulver mit einer mittleren Korngröße von kleiner etwa 100 µm verwendet. Ein geeignetes PEEK-Pulver ist beispielsweise unter der Bezeichnung VESTAKEEP^{®} 2000 FP im Handel verfügbar. Dieses weist eine durchschnittliche Korngröße von etwa 50 µm und eine Schmelze-Volumenfließrate (MVR) von 70 cm³/10 min (Prüftemperatur 380 °C, Masse 5 kg) auf.

Es kann auch vorgesehen sein, dass die Mischung ein flockenförmiges Polyaryletherketon umfasst. Hierbei kann die Form der Flocken durchaus von der sphärischen Form abweichen. Vorzugsweise haben die Flocken einen Durchmesser von weniger als etwa 5000 µm und eine Dicke von weniger als etwa 200 µm.

Gemäß einer besonders bevorzugten Ausführungsvariante kann vorgesehen sein, dass pulverförmiges Polyetheretherketon mit Natriumchlorid vermengt wird, wobei vorzugsweise der Massenanteil des pulverförmigen Polyetheretherketons in der Mischung etwa 4 % bis 65 %, besonders bevorzugt etwa 5 % bis 55 %, ganz besonders bevorzugt etwa 6 % bis 45 %, beträgt, wobei vorzugsweise eine durchschnittliche Korngröße des pulverförmigen Polyetheretherketons kleiner etwa 1000 µm, besonders bevorzugt kleiner etwa 100 µm, ist.

Vorzugsweise kann vorgesehen sein, dass die Mischung auf eine Maximaltemperatur zwischen etwa 335 °C und etwa 450 °C, vorzugsweise auf etwa 370 °C, erhitzt wird, wobei vorzugsweise die Mischung während einer Verweildauer von bis zu 4 h, besonders bevorzugt etwa 0,5 h bis 1,5 h, der Maximaltemperatur ausgesetzt wird.

Da die Schmelztemperatur von PEEK 335 °C und die Zersetzungstemperatur etwa 450 °C beträgt, sollte die Mischung auf eine Maximaltemperatur überhalb der Schmelztemperatur von PEEK und unterhalb der Zersetzungstemperatur von PEEK erhitzt werden. Versuche der Anmelderin haben ergeben, dass eine Maximaltemperatur von etwa 370 °C zu einer gut weiterverarbeitbaren Schmelze führt.

Vorzugsweise ist die Erhitzung der Mischung derart hoch und/oder lang, dass die Mischung zumindest oberflächlich anschmilzt oder sich eine Schmelze bildet. Die Verweilzeit während der Erhitzung kann je nach Weiterverarbeitungsmethode von nur etwa 1 s (z.B. bei einem kontinuierlichen Verfahren) bis etwa 4 h betragen.

Das Formen der Verdampferkörper kann beispielsweise über die folgenden an sich bekannten Formgebungsverfahren erfolgen: Batch (flächige Herstellung, die Formen werden später gebohrt, gestanzt oder geschnitten; mit oder ohne integralem Heizelement), Extrusion (als Schnur oder Schlauch; mit oder ohne integralem Heizelement), Sintern oder Spritzguss.

Wenn der Mischung vor dem Erhitzen und Formen ein löslicher Feststoff (wie zum Beispiel ein Salz) beigemengt war, kann es vorgesehen sein, dass nach dem Formen des Verdampferkörpers und gegebenenfalls nach dem Abkühlen des Verdampferkörpers der lösliche Feststoff mit einem geeigneten Lösungsmittel (z.B. Wasser) so weit wie möglich aus dem Verdampferkörper herausgelöst wird, um die gewünschte Porosität des Verdampferkörpers zu erhalten. So kann der Verdampferkörper eine offene Porosität von etwa 30 % bis 96 %, vorzugsweise von etwa 50 % bis 95 %, besonders bevorzugt von etwa 65 % bis 94 %, aufweisen.

Nachfolgend sind Beispiele für gemäß dem vorgeschlagenen Verfahren hergestellte Verdampferkörper angegeben.

### Beispiel 1:

Die Mischung enthält PEEK mit einem Massenanteil von 8 % und NaCl mit einem Massenanteil von 92 %. Der Partikeldurchmesser des PEEK-Pulvers bewegt sich von etwa 4 µm bis etwa 832 µm, mit einem mittleren Partikeldurchmesser von etwa 70 µm. Der Partikeldurchmesser des NaCl-Pulvers bewegt sich von etwa 189 µm bis etwa 1200 µm, mit einem mittleren Partikeldurchmesser von etwa 479 µm. Die Mischung wird in einem Ofen erhitzt. Die Ofentemperatur beträgt etwa 370 °C und die Ofenverweilzeit beträgt etwa 1 h. Nach dem Abkühlen und Erstarren der Schmelze wird das NaCl mit Wasser herausgelöst. Es ergibt sich eine offene Porosität von ca. 91 %.

### Beispiel 2:

Die Mischung enthält PEEK mit einem Massenanteil von 20 % und NaCl mit einem Massenanteil von 80 %. Der Partikeldurchmesser des PEEK-Pulvers bewegt sich von etwa 4 µm bis etwa 832 µm, mit einem mittleren Partikeldurchmesser von etwa 70 µm. Der Partikeldurchmesser des NaCl-Pulvers bewegt sich von etwa 132 µm bis etwa 692 µm, mit einem mittleren Partikeldurchmesser von etwa 293 µm. Die Mischung wird in einem Ofen erhitzt. Die Ofentemperatur beträgt etwa 370 °C und die Ofenverweilzeit beträgt etwa 1 h. Nach dem Abkühlen und Erstarren der Schmelze wird das NaCl mit Wasser herausgelöst. Es ergibt sich eine offene Porosität von ca. 73 %.

### Beispiel 3:

Die Mischung enthält PEEK mit einem Massenanteil von 15 % und NaCl mit einem Massenanteil von 85 %. Der Partikeldurchmesser des PEEK-Pulvers bewegt sich von etwa 4 µm bis etwa 832 µm, mit einem mittleren Partikeldurchmesser von etwa 70 µm. Der Partikeldurchmesser des NaCl-Pulvers bewegt sich von etwa 0,9 µm bis etwa 437 µm, mit einem mittleren Partikeldurchmesser von etwa 293 µm. Die Mischung wird in einem Ofen erhitzt. Die Ofentemperatur beträgt etwa 370 °C und die Ofenverweilzeit beträgt etwa 1 h. Nach dem Abkühlen und Erstarren der Schmelze wird das NaCl mit Wasser herausgelöst. Es ergibt sich eine offene Porosität von ca. 85 %.

### Beispiel 4:

Die Mischung enthält PEEK mit einem Massenanteil von 23 %, Kieselgur mit einem Massenanteil von 23 % und H₂O mit einem Massenanteil von 54 %. Der Partikeldurchmesser des PEEK-Pulvers bewegt sich von etwa 4 µm bis etwa 832 µm, mit einem mittleren Partikeldurchmesser von etwa 70 µm. Der Partikeldurchmesser des Kieselgur-Pulvers bewegt sich von etwa 0,8 µm bis etwa 331 µm, mit einem mittleren Partikeldurchmesser von etwa 9 µm. Die Mischung wird zunächst während einer Dauer von 12 h auf 105 °C erhitzt, wodurch die Mischung getrocknet wird. Anschließend wird die Erhitzung der Mischung in einem Ofen fortgesetzt. Die Ofentemperatur beträgt etwa 370 °C und die Ofenverweilzeit beträgt etwa 1 h. Es ergibt sich eine offene Porosität von ca. 85 %.

### Beispiel 5:

Die Mischung enthält PEEK mit einem Massenanteil von 40 % und Graphit mit einem Massenanteil von 60 %. Der Partikeldurchmesser des PEEK-Pulvers bewegt sich von etwa 4 µm bis etwa 832 µm, mit einem mittleren Partikeldurchmesser von etwa 70 µm. Der Partikeldurchmesser des Graphit-Pulvers bewegt sich von etwa 1 µm bis etwa 631 µm, mit einem mittleren Partikeldurchmesser von etwa 18 µm. Die Mischung wird in einem Ofen erhitzt. Die Ofentemperatur beträgt etwa 370 °C und die Ofenverweilzeit beträgt etwa 1 h. Es ergibt sich eine offene Porosität von ca. 57 %.

Schutz wird auch begehrt für ein Verfahren zur Herstellung eines Verdampferkörpers mit den Merkmalen des Anspruchs 21. Vorteilhafte Ausführungsformen sind in den davon abhängigen Ansprüchen angegeben.

Beim vorgeschlagenen Verfahren wird bzw. werden wenigstens eine Faser und/oder wenigstens ein Filament umfassend den wenigstens einen thermoplastischen Kunststoff zu einem textilen Gebilde, vorzugsweise zu einem flächigen oder räumlichen Gewebe, verarbeitet, wobei aus dem textilen Gebilde der Verdampferkörper oder ein Teil davon geformt wird.

Es kann vorgesehen sein, dass ein Faden und/oder Garn umfassend die wenigstens eine Faser und/oder das wenigstens eine Filament zum textilen Gebilde verarbeitet wird bzw. werden.

Vorzugsweise kann vorgesehen sein, dass die Verarbeitung unter Verwendung wenigstens eines der folgenden Verfahren erfolgt: Spinnen, Weben, Wirken, Stricken, Flechten, Filzen, Fügen.

Es kann vorgesehen sein, dass beim Schritt des Formens des Verdampferkörpers das textile Gebilde mit einer temperierten Klinge zugeschnitten wird.

Generell kann gemäß einer bevorzugten Ausführungsform vorgesehen sein, dass auf eine Oberfläche des Verdampferkörpers wenigstens bereichsweise wenigstens eine elektrisch leitfähige Beschichtung aufgebracht wird. Die wenigstens eine elektrisch leitfähige Beschichtung kann als Heizvorrichtung dienen.

Vorzugsweise kann vorgesehen sein, dass die wenigstens eine elektrisch leitfähige Beschichtung galvanisch auf die Oberfläche des Verdampferkörpers aufgebracht wird. Dies kann unter Einsatz von an sich bekannter Galvanotechnik erfolgen. Im Verdampferkörper allenfalls vorhandene Metallpulver-Partikel können dabei als "Keime" für das Galvanisieren dienen.

Es kann auch vorgesehen sein, dass die wenigstens eine elektrisch leitfähige Beschichtung auf die Oberfläche des Verdampferkörpers aufgedampft wird.

Weitere Einzelheiten und Vorteile der vorliegenden Erfindung werden anhand der nachfolgenden Figurenbeschreibung erläutert. Dabei zeigt bzw. zeigen:
- Fig. 1: ein Ausführungsbeispiel eines Inhalators in Form einer Elektrozigarette in einer Frontansicht,
- Fig. 2: eine Schnittdarstellung durch eine Elektrozigarette gemäß Figur 1,
- Fig. 3: eine vergrößerte Detailansicht eines Bereichs der Figur 2,
- Fig. 4: eine vergrößerte Detailansicht eines Bereichs der Figur 3,
- Fig. 5: eine Seitenansicht auf den Verdampferkörper der Figur 4,
- Fig. 6: eine schematische Darstellung gemäß Figur 4,
- Fig. 7: ein weiteres Ausführungsbeispiel eines Inhalators in Form einer Elektrozigarette in einer Frontansicht,
- Fig. 8: eine Schnittdarstellung durch eine Elektrozigarette gemäß Figur 7,
- Fig. 9: eine vergrößerte Detailansicht eines Bereichs der Figur 8,
- Fig. 10: eine vergrößerte Detailansicht eines Bereichs der Figur 9,
- Fig. 11: eine Seitenansicht auf den Verdampferkörper der Figur 10,
- Fig. 12: eine schematische Darstellung gemäß Figur 9,
- Fig. 13 - 17: Ausführungsbeispiele von vorgeschlagenen Verdampferkörpern,
- Fig. 18: ein Ausführungsbeispiel einer Verdampfervorrichtung und
- Fig. 19: ein Ausführungsbeispiel eines als textiles Gebilde ausgebildeten Verdampferkörpers.

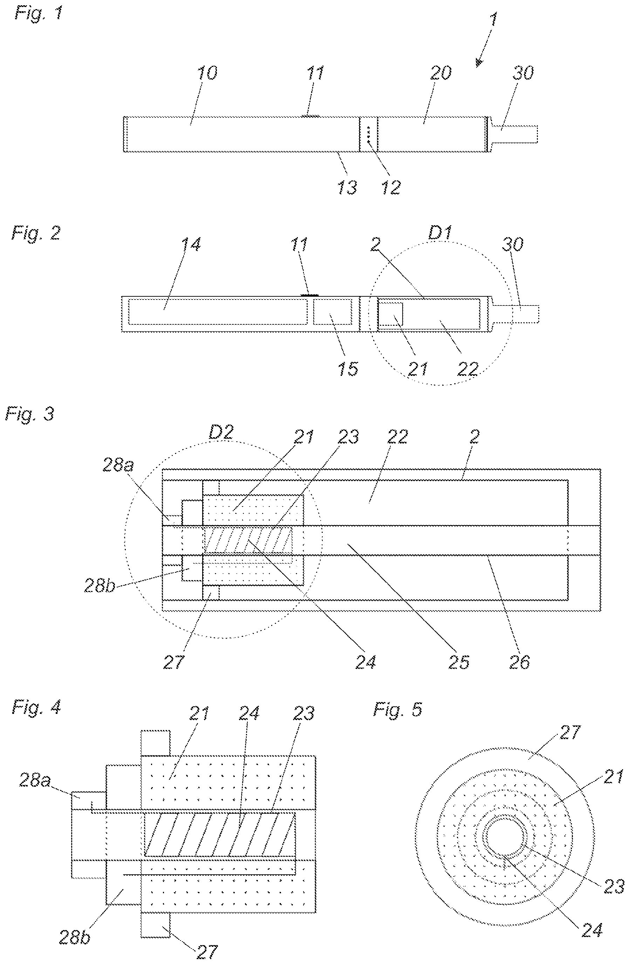

Figur 1 zeigt ein Ausführungsbeispiel eines Inhalators in Form einer Elektrozigarette 1 in einer Frontansicht.

Die Elektrozigarette 1 umfasst ein erstes Gehäuseteil 10, in dem ein in dieser Darstellung nicht ersichtlicher Energiespeicher in Form eines Akkumulators 14 sowie eine hier ebenfalls nicht sichtbare Steuerungselektronik 15 der Elektrozigarette 1 untergebracht sind. Ferner sind am ersten Gehäuseteil 10 ein Bedienelement 11 in Form eines Tastschalters zum Betätigen der Elektrozigarette 1 und ein Ladeanschluss 13 zum Aufladen des Akkumulators 14 angebracht. Das erste Gehäuseteil 10 weist zudem einen Lufteinlass 12 (hier in Form von mehreren übereinander angeordneten Löchern im Gehäuse) auf, über den Umgebungsluft in das Innere der Elektrozigarette 1 eingebracht werden kann, indem an einem Mundstück 30 der Elektrozigarette 1 gesaugt wird.

Die Elektrozigarette 1 umfasst darüber hinaus ein zweites Gehäuseteil 20, das mit dem ersten Gehäuseteil 10 verschraubbar ist. Im zweiten Gehäuseteil 20 ist eine in dieser Darstellung nicht ersichtliche Verdampfervorrichtung 2 zum Verdampfen eines hier ebenfalls nicht ersichtlichen Liquids untergebracht. Am zweiten Gehäuseteil 20 ist ein Mundstück 30 angeordnet, über das bei aktivierter Elektrozigarette 1 verdampftes Liquid entnehmbar ist.

Figur 2 zeigt eine schematische Schnittdarstellung durch eine Elektrozigarette 1 gemäß Figur 1. In dieser Darstellung sind insbesondere der Akkumulator 14 und die Steuerungselektronik 15 ersichtlich, die innerhalb des ersten Gehäuseteils 10 angeordnet sind. Ebenfalls ersichtlich ist die im zweiten Gehäuseteil 20 angeordnete Verdampfervorrichtung 2. Die Verdampfervorrichtung 2 umfasst einen Verdampferkörper 21 und einen Aufnahmeraum 22 in Form eines Tanks, der zu verdampfendes Liquid enthält. Der Verdampferkörper 21 ragt in den Aufnahmeraum 22 und steht mit dem darin vorhandenen Liquid in Verbindung.

Figur 3 zeigt eine vergrößerte Detailansicht des in Figur 2 markierten und mit D1 bezeichneten Bereichs.

Der Verdampferkörper 21 ragt in den das Liquid enthaltende Aufnahmeraum 22 und begrenzt gemeinsam mit einer um einen Endbereich des Verdampferkörpers 21 angeordneten, umlaufenden Dichtung 27 den Aufnahmeraum 22. Der Verdampferkörper 21 ist selbsttragend und besteht in diesem Beispiel im Wesentlichen vollständig aus PEEK. Der Verdampferkörper 21 weist eine offene Porosität von etwa 80 % auf.

Durch Verdampferkörper 21 und Aufnahmeraum 22 verläuft ein rohrförmiger Kanal 26, der ebenfalls den Aufnahmeraum 22 begrenzt, wodurch das Liquid nicht in das Innere des Kanals 26 strömen kann. Der Verdampferkörper 21 ist in diesem Beispiel kreiszylindrisch ausgebildet und ein Abschnitt des Kanals 26 ist im Verdampferkörper 21 als ein den Verdampferkörper 21 in einer Längsrichtung durchsetzendes Durchgangsloch 23 ausgebildet. Im Inneren des Verdampferkörpers 21, in diesem Beispiel innerhalb des den Verdampferkörper 21 durchsetzenden Durchgangslochs 23, ist eine Heizvorrichtung 24 in Form einer Heizwendel angeordnet. Die Heizvorrichtung 24 ist über elektrische Kontaktierungen 28a (z.B. ein positiver Spannungsanschluss) und 28b (z.B. ein negativer Spannungsanschluss) mit der Steuerungselektronik 15 verbunden. Bei Betätigung der Elektrozigarette 1 über das Bedienelement 11 wird über die Steuerungselektronik 15 Strom aus dem Akkumulator 14 über die elektrischen Kontaktierungen 28a, 28b in die Heizvorrichtung 24 geleitet, wodurch sich die Heizvorrichtung 24 erhitzt. Durch die Porosität des Verdampferkörpers 21 gelangt Liquid, mit dem der Verdampferkörper 21 in Verbindung steht, in den Verdampferkörper 21. Das Erhitzen der Heizvorrichtung 24 führt zu einer Verdampfung des Liquids im Verdampferkörper 21. Durch Saugen des Benutzers der Elektrozigarette 1 am Mundstück 30 wird Luft von außerhalb der Elektrozigarette 1 durch den Lufteinlass 12 in das Innere der Elektrozigarette 1 eingesaugt. Die Luft strömt dabei durch den Kanal 26, wobei die Luft das verdampfte Liquid aufnimmt. Das Gemisch 25 aus Luft und verdampftem Liquid strömt weiter durch den Kanal 26 und verlässt die Elektrozigarette 1 durch das Mundstück 30.

Figur 4 zeigt eine vergrößerte Detailansicht des in Figur 3 markierten und mit D2 bezeichneten Bereichs. Hier ist deutlich das Durchgangsloch 23 im Verdampfungskörper 21 ersichtlich, welches einen Abschnitt des Kanals 26 bildet. In dem Durchgangsloch 23 ist die Heizvorrichtung 24 angeordnet und mit zwei elektrischen Kontaktierungen 28a, 28b elektrisch kontaktiert. An einem Endbereich des Verdampferkörpers 21 ist eine umlaufende Dichtung 27 angeordnet, sodass bei Einsetzen des Verdampferkörpers 21 mit umlaufender Dichtung 27 in den Aufnahmeraum 22 der Aufnahmeraum 22 stirnseitig begrenzt und abgedichtet ist.

Figur 5 zeigt eine Seitenansicht auf den Verdampferkörper 21 der Figur 4.

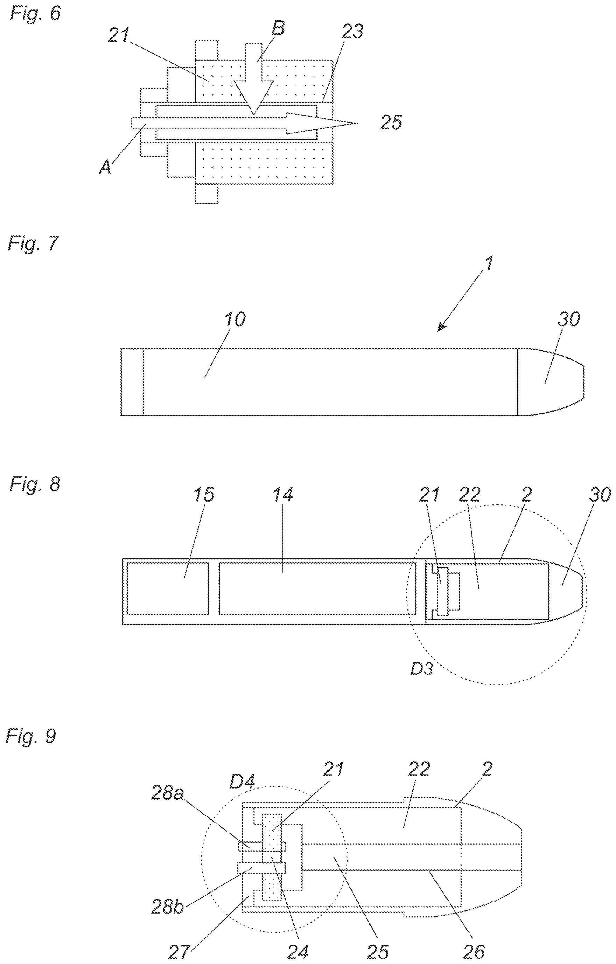

Figur 6 zeigt eine schematische Darstellung gemäß Figur 4, in der insbesondere die Strömung von Luft A, verdampftem Liquid B und Gemisch 25 aus Luft A und verdampftem Liquid B durch das Durchgangsloch 23 des Verdampferkörpers 21 dargestellt ist.

Figur 7 zeigt ein weiteres Ausführungsbeispiel eines Inhalators in Form einer Elektrozigarette 1 in einer Frontansicht. Die Elektrozigarette 1 besteht aus einem ersten Gehäuseteil 10 und einem in das erste Gehäuseteil 10 einschiebbaren Mundstück 30. Am ersten Gehäuseteil 10 ist ein hier nicht ersichtlicher Lufteinlass 12 angeordnet, über den bei Betätigung der Elektrozigarette 1 Luft aus der Umgebung in das Innere der Elektrozigarette 1 gelangen kann.

Figur 8 zeigt eine schematische Schnittdarstellung durch eine Elektrozigarette 1 gemäß Figur 7. In dieser Darstellung sind insbesondere der Akkumulator 14 und die Steuerungselektronik 15 ersichtlich, die innerhalb des ersten Gehäuseteils 10 angeordnet sind. Ebenfalls ersichtlich ist die im abnehmbaren Mundstück 30 angeordnete Verdampfervorrichtung 2. Die Verdampfervorrichtung 2 umfasst einen Verdampferkörper 21 und einen Aufnahmeraum 22 in Form eines Tanks, der zu verdampfendes Liquid enthält. Der Verdampferkörper 21 ist im Wesentlichen kreiszylindrisch ausgebildet und ragt mit beiden Endbereichen in den Aufnahmeraum 22, sodass er mit beiden Endbereichen mit dem im Aufnahmeraum 22 vorhandenen Liquid in Verbindung steht.

Figur 9 zeigt eine vergrößerte Detailansicht des in Figur 8 markierten und mit D3 bezeichneten Bereichs.

Der Verdampferkörper 21 ist selbsttragend und besteht in diesem Beispiel im Wesentlichen vollständig aus PEEK. Der Verdampferkörper 21 weist eine offene Porosität von etwa 75 % auf. Der Verdampferkörper 21 ragt mit beiden Endbereichen in den das Liquid enthaltende Aufnahmeraum 22. Aufgrund der Porosität des Verdampferkörpers 21 kann somit Liquid vom Aufnahmeraum 22 in die Hohlräume des Verdampferkörpers 21 eindringen. Der Verdampferkörper 21 ist an einer Dichtung 27 in Form eines Dichtungsdeckels angeordnet, der den Aufnahmeraum 22 begrenzt.

Durch den Aufnahmeraum 22 verläuft ein rohrförmiger Kanal 26, der ebenfalls den Aufnahmeraum 22 begrenzt, wodurch das Liquid nicht in das Innere des Kanals 26 strömen kann. Der Verdampferkörper 21 ist vor einer Mündung des Kanals 26 angeordnet. Am Verdampferkörper 21 ist eine Heizvorrichtung 24 in Form einer Heizwendel angeordnet, die in diesem Beispiel um einen Abschnitt des Verdampferkörpers 21 herumgewickelt ist. Die Heizvorrichtung 24 ist über elektrische Kontaktierungen 28a (z.B. ein positiver Spannungsanschluss) und 28b (z.B. ein negativer Spannungsanschluss) mit der Steuerungselektronik 15 verbunden.

Die Elektrozigarette 1 dieses Beispiels ist über einen nicht dargestellten Unterdrucksensor, der mit der Steuerungselektronik 15 verbunden ist, aktivierbar. Durch Saugen des Benutzers der Elektrozigarette 1 am Mundstück 30 wird Luft von außerhalb der Elektrozigarette 1 durch den Lufteinlass 12 in das Innere der Elektrozigarette 1 eingesaugt. Darüber hinaus entsteht beim Saugen am Mundstück 30 im Inneren der Elektrozigarette 1 ein Unterdruck, der vom Unterdrucksensor detektiert wird, woraufhin die Elektrozigarette 1 von der Steuerungselektronik 15 aktiviert wird. Dabei wird über die Steuerungselektronik 15 Strom aus dem Akkumulator 14 über die elektrischen Kontaktierungen 28a, 28b in die Heizvorrichtung 24 geleitet, wodurch sich die Heizvorrichtung 24 erhitzt. Durch die Porosität des Verdampferkörpers 21 gelangt Liquid, mit dem der Verdampferkörper 21 in Verbindung steht, in den Verdampferkörper 21. Das Erhitzen der Heizvorrichtung 24 führt zu einer Verdampfung des Liquids im Verdampferkörper 21. Die eingesaugte Luft strömt durch bzw. um den Verdampferkörper 21, wobei die Luft das verdampfte Liquid aufnimmt. Das Gemisch 25 aus Luft und verdampftem Liquid strömt in die Mündung des Kanals 26 und weiter durch den Kanal 26 und verlässt die Elektrozigarette 1 durch das Mundstück 30.

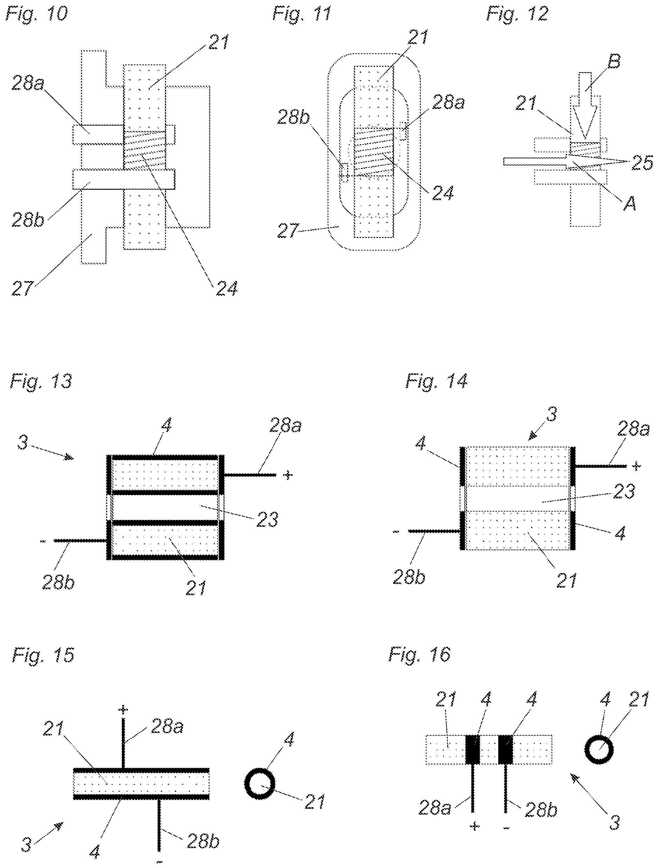

Figur 10 zeigt eine vergrößerte Detailansicht des in Figur 9 markierten und mit D4 bezeichneten Bereichs. Hier ist deutlich der Verdampferkörper 21 ersichtlich, um den die Heizvorrichtung 24 in Form einer Heizwendel herumgewickelt ist. Die Heizvorrichtung 24 ist mit zwei elektrischen Kontaktierungen 28a, 28b elektrisch kontaktiert, die bis zur Dichtung 27 in Form eines Dichtungsdeckels geführt sind und für eine Vereinfachung der Kontaktierung leicht über den Dichtungsdeckel hinausragen.

Die Form des Dichtungsdeckels korrespondiert mit der Form einer Stirnseite des Aufnahmeraums 22, sodass bei Einsetzen des Dichtungsdeckels auf den Aufnahmeraum 22 der Aufnahmeraum 22 stirnseitig begrenzt und abgedichtet ist.

Figur 11 zeigt eine Seitenansicht auf den Verdampferkörper 21 der Figur 10.

Figur 12 zeigt eine schematische Darstellung eines Verdampferkörpers 21 gemäß Figur 9, in der insbesondere die Strömung von Luft A, verdampftem Liquid B und Gemisch 25 aus Luft A und verdampftem Liquid B dargestellt ist.

Figur 13 zeigt einen vorgeschlagenen Verdampferkörper 21 in einem Längsschnitt. Der Verdampferkörper 21 ist kreiszylindrisch ausgebildet und von einem Durchgangsloch 23 durchsetzt, sodass der Verdampferkörper 21 insgesamt die Gestalt eines Rundrohres annimmt. In diesem Beispiel ist die gesamte Oberfläche 3 des Verdampferkörpers 21 - also die Innenwandung des Durchgangslochs 23, die Mantelfläche des Verdampferkörpers 21 und die Stirnflächen des Verdampferkörpers 21 - mit einer elektrisch leitfähigen Beschichtung 4 versehen. Die elektrisch leitfähige Beschichtung 4 stellt in diesem Beispiel eine Heizvorrichtung 24 dar, die über elektrische Kontaktierungen 28a (z.B. ein positiver Spannungsanschluss) und 28b (z.B. ein negativer Spannungsanschluss) mit einer entsprechenden Spannungsversorgung verbunden werden kann.

Figur 14 zeigt ein weiteres Ausführungsbeispiel eines vorgeschlagenen Verdampferkörpers 21 in einem Längsschnitt. Auch dieser Verdampferkörper 21 ist rundrohrförmig ausgebildet, allerdings ist nicht die gesamte Oberfläche 3 des Verdampferkörpers 21 mit einer elektrisch leitfähigen Beschichtung 4 versehen. Lediglich die Stirnseiten des Verdampferkörpers 21 sind mit jeweils einer elektrisch leitfähigen Beschichtung 4 versehen. In diesem Beispiel umfasst das Material des Verdampferkörpers 21 einen elektrisch leitfähigen Zusatzstoff, sodass der Verdampferkörper 21 in sich elektrisch leitfähig ist. Somit bildet der Verdampferkörper 21 selbst in Verbindung mit den beiden elektrisch leitfähigen Beschichtungen 4 eine Heizvorrichtung 24, die über elektrische Kontaktierungen 28a, 28b mit einer Spannungsversorgung verbunden werden kann.

Die Verdampferkörper 21 gemäß den Figuren 13 und 14 eignen sich insbesondere für den Einsatz in einer Elektrozigarette 1 gemäß Figur 1.

Figur 15 zeigt ein Ausführungsbeispiel eines Verdampferkörpers 21 in einem Längsschnitt und in einer Seitenansicht. Der Verdampferkörper 21 ist im Wesentlichen kreiszylindrisch ausgebildet. In diesem Beispiel ist die Oberfläche 3 des Verdampferkörpers 21 bereichsweise mit einer elektrisch leitfähigen Beschichtung 4 versehen. Konkret ist die Mantelfläche des kreiszylindrischen Verdampferkörpers 21 mit einer elektrisch leitfähigen Beschichtung 4 versehen. Die elektrisch leitfähige Beschichtung 4 stellt in diesem Beispiel eine Heizvorrichtung 24 dar, die über elektrische Kontaktierungen 28a, 28b mit einer Spannungsversorgung verbunden werden kann.

Figur 16 zeigt ein weiteres Ausführungsbeispiel eines Verdampferkörpers 21 in einem Längsschnitt und in einer Seitenansicht. Der Verdampferkörper 21 ist im Wesentlichen kreiszylindrisch ausgebildet. Das Material des Verdampferkörpers 21 umfasst einen elektrisch leitfähigen Zusatzstoff, sodass der Verdampferkörper 21 in sich elektrisch leitfähig ist. An der Mantelfläche des Verdampferkörpers 21 sind zwei voneinander getrennte, umlaufende elektrisch leitfähige Beschichtungen 4 angebracht. Der in sich elektrisch leitfähige Verdampferkörper 21 bildet in Verbindung mit den beiden elektrisch leitfähigen Beschichtungen 4 eine Heizvorrichtung 24, die über elektrische Kontaktierungen 28a, 28b mit einer Spannungsversorgung verbunden werden kann.

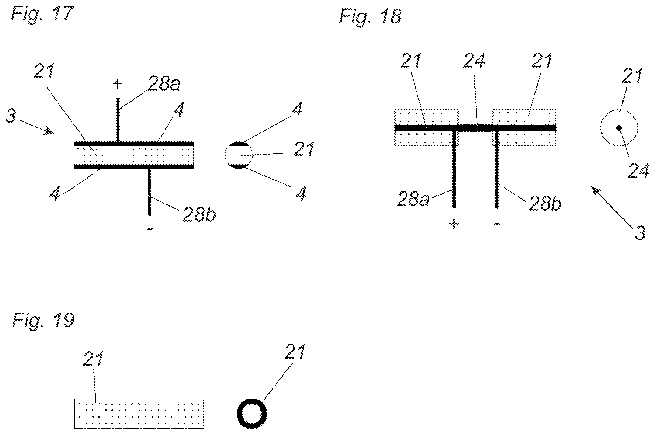

Figur 17 zeigt ein weiteres Ausführungsbeispiel eines Verdampferkörpers 21 in einem Längsschnitt und in einer Seitenansicht. Der Verdampferkörper 21 ist im Wesentlichen kreiszylindrisch ausgebildet. Das Material des Verdampferkörpers 21 umfasst einen elektrisch leitfähigen Zusatzstoff, sodass der Verdampferkörper 21 in sich elektrisch leitfähig ist. An der Mantelfläche des Verdampferkörpers 21 sind zwei voneinander getrennte, entlang einer Längserstreckung des Verdampferkörpers 21 verlaufende elektrisch leitfähige Beschichtungen 4 angebracht. Der in sich elektrisch leitfähige Verdampferkörper 21 bildet in Verbindung mit den beiden elektrisch leitfähigen Beschichtungen 4 eine Heizvorrichtung 24, die über elektrische Kontaktierungen 28a, 28b mit einer Spannungsversorgung verbunden werden kann.

Figur 18 zeigt ein Ausführungsbeispiel einer Verdampfervorrichtung 2 in einem Längsschnitt und in einer Seitenansicht. Die Verdampfervorrichtung 2 umfasst zwei in Längsrichtung voneinander beabstandete, kreiszylindrisch ausgebildete Verdampferkörper 21 und eine Heizvorrichtung 24 in Form eines elektrisch leitfähigen Drahtes, der in beide Verdampferkörper 21 integriert ist und beide Verdampferkörper 21 miteinander verbindet. Die Heizvorrichtung 24 kann über elektrische Kontaktierungen 28a, 28b mit einer Spannungsversorgung verbunden werden.

Figur 19 zeigt ein weiteres Ausführungsbeispiel eines Verdampferkörpers 21 in einer Frontansicht und in einer Seitenansicht. Der Verdampferkörper 21 ist in diesem Beispiel als textiles Gebilde in Form eines PEEK-Gewebes ausgebildet. Der Verdampferkörper 21 hat einen dochtartigen Aufbau, wodurch er ein Liquid für eine Elektrozigarette optimal aufnehmen und weiter transportieren kann. Das PEEK-Gewebe ist in diesem Beispiel aus PEEK-Filament mit einem Durchmesser von etwa 38 µm gewoben worden. Die runde Geometrie wurde mittels üblicher Webverfahren hergestellt.

Die Verdampferkörper 21 gemäß den Figuren 15 bis 17 und 19 und die Verdampfervorrichtung 2 gemäß der Figur 18 eignen sich insbesondere für den Einsatz in einer Elektrozigarette 1 gemäß Figur 7.

## Patentansprüche

1. Verdampferkörper (21) für eine Verdampfervorrichtung (2) eines Inhalators, insbesondere einer Elektrozigarette (1), wobei der Verdampferkörper (21) ein Liquid aufnehmen kann und aus einem Material besteht, das wenigstens einen thermoplastischen Kunststoff umfasst, **dadurch gekennzeichnet, dass** das Material des Verdampferkörpers (21) wenigstens einen elektrisch leitfähigen Zusatzstoff umfasst, der mit dem wenigstens einen thermoplastischen Kunststoff zur Bereitstellung einer Mischung vermengt ist.

2. Verdampferkörper nach Anspruch 1, **dadurch gekennzeichnet, dass** eine Schmelztemperatur des wenigstens einen thermoplastischen Kunststoffs größer etwa 180 °C, vorzugweise größer etwa 250 °C, beträgt oder eine Glasübergangstemperatur des wenigstens einen thermoplastischen Kunststoffs größer etwa 80 °C, vorzugweise größer etwa 150 °C, beträgt.

3. Verdampferkörper nach einem der Ansprüche 1 bis 2, **dadurch gekennzeichnet, dass** der Verdampferkörper (21) im Wesentlichen vollständig aus wenigstens einem Polyaryletherketon, vorzugsweise Polyetheretherketon, besteht.

4. Verdampferkörper nach einem der Ansprüche 1 bis 3, **dadurch gekennzeichnet, dass** der Verdampferkörper (21) eine offene Porosität von etwa 30 % bis 96 %, vorzugsweise von etwa 50 % bis 95 %, besonders bevorzugt von etwa 65 % bis 94 %, aufweist.

5. Verdampferkörper nach einem der Ansprüche 1 bis 4, **dadurch gekennzeichnet, dass** das Material des Verdampferkörpers (21) wenigstens ein Additiv umfasst.

6. Verdampferkörper nach einem der Ansprüche 1 bis 5, **dadurch gekennzeichnet, dass** der wenigstens eine elektrisch leitfähige Zusatzstoff ein Metall, Graphit, Graphen, Siliciumcarbid oder Aktivkohle ist.

7. Verdampferkörper nach einem der Ansprüche 1 bis 6, **dadurch gekennzeichnet, dass** eine Oberfläche (3) des Verdampferkörpers (21) wenigstens bereichsweise mit wenigstens einer elektrisch leitfähigen Beschichtung (4) versehen ist.

8. Verdampferkörper nach einem der Ansprüche 1 bis 7, **dadurch gekennzeichnet, dass** der Verdampferkörper (21) zumindest bereichsweise, vorzugsweise im Wesentlichen vollständig, als textiles Gebilde, vorzugsweise als flächiges oder räumliches Gewebe, ausgebildet ist, wobei das textile Gebilde wenigstens eine Faser und/oder wenigstens ein Filament umfassend den wenigstens einen thermoplastischen Kunststoff umfasst.

9. Verdampferkörper nach Anspruch 8, **dadurch gekennzeichnet, dass** die wenigstens eine Faser und/oder das wenigstens eine Filament im Wesentlichen vollständig aus wenigstens einem Polyaryletherketon, vorzugsweise Polyetheretherketon, besteht bzw. bestehen, und/oder dass die wenigstens eine Faser und/oder das wenigstens eine Filament einen Durchmesser kleiner 1000 µm, vorzugsweise kleiner 500 µm, besonders bevorzugt kleiner 300 µm, aufweist bzw. aufweisen.

10. Verdampfervorrichtung (2) für einen Inhalator, insbesondere eine Elektrozigarette (1), mit wenigstens einem Verdampferkörper (21) nach einem der Ansprüche 1 bis 9.

11. Verdampfervorrichtung nach Anspruch 10, **dadurch gekennzeichnet, dass** die Verdampfervorrichtung (2) einen Aufnahmeraum (22) für ein Liquid umfasst, wobei der Verdampferkörper (21) mit dem Liquid in Verbindung steht.

12. Inhalator, insbesondere Elektrozigarette (1), mit wenigstens einer Verdampfervorrichtung (2) nach einem der Ansprüche 10 bis 11.

13. Mundstück (30) für einen Inhalator, insbesondere eine Elektrozigarette (1), mit wenigstens einer Verdampfervorrichtung (2) nach einem der Ansprüche 10 bis 11.

14. Verfahren zur Herstellung eines Verdampferkörpers (21) nach einem der Ansprüche 1 bis 9, **dadurch gekennzeichnet, dass** eine Mischung umfassend wenigstens einen thermoplastischen Kunststoff bereitgestellt wird, wobei die Mischung erhitzt wird bis die Mischung zumindest oberflächlich geschmolzen ist oder sich eine Schmelze bildet, wobei aus der zumindest oberflächlich geschmolzen Mischung oder der Schmelze wenigstens ein Verdampferkörper (21) geformt wird, wobei die Mischung wenigstens einen elektrisch leitfähigen Zusatzstoff umfasst, der mit dem wenigstens einen thermoplastischen Kunststoff zur Bereitstellung der Mischung vermengt wird.

15. Verfahren nach Anspruch 13 oder 14, **dadurch gekennzeichnet, dass** die Mischung wenigstens ein Additiv umfasst, das mit dem wenigstens einen thermoplastischen Kunststoff zur Bereitstellung der Mischung vermengt wird.

16. Verfahren nach Anspruch 15, **dadurch gekennzeichnet, dass** die Mischung wenigstens einen löslichen Feststoff, vorzugweise ein Salz umfasst, wobei der wenigstens eine lösliche Feststoff mit dem wenigstens einen thermoplastischen Kunststoff zur Bereitstellung der Mischung vermengt wird, wobei nach dem Formen des wenigstens einen Verdampferkörpers (21) der wenigstens eine lösliche Feststoff zumindest teilweise, vorzugsweise im Wesentlichen vollständig, aus dem Verdampferkörper (21) herausgelöst wird.

17. Verfahren nach einem der Ansprüche 13 bis 16, **dadurch gekennzeichnet, dass** der wenigstens eine thermoplastische Kunststoff ein pulver- oder flockenförmiges Polyaryletherketon, vorzugsweise Polyetheretherketon, ist.

18. Verfahren nach Anspruch 17, **dadurch gekennzeichnet, dass** der Massenanteil des pulver- oder flockenförmigen Polyaryletherketons in der Mischung etwa 4 % bis 65 %, vorzugsweise etwa 5 % bis 55 %, besonders bevorzugt etwa 6 % bis 45 % beträgt.

19. Verfahren nach Anspruch 17 oder 18, **dadurch gekennzeichnet, dass** pulverförmiges Polyetheretherketon mit Natriumchlorid vermengt wird, wobei vorzugsweise der Massenanteil des pulverförmigen Polyetheretherketons in der Mischung etwa 4 % bis 65 %, besonders bevorzugt etwa 5 % bis 55 %, ganz besonders bevorzugt etwa 6 % bis 45 %, beträgt, wobei vorzugsweise eine durchschnittliche Korngröße des pulverförmigen Polyetheretherketons kleiner etwa 1000 µm, besonders bevorzugt kleiner etwa 100 µm, ist.

20. Verfahren nach einem der Ansprüche 14 bis 19, **dadurch gekennzeichnet, dass** die Mischung auf eine Maximaltemperatur zwischen etwa 350 °C und etwa 450 °C, vorzugsweise auf etwa 370 °C, erhitzt wird, wobei vorzugsweise die Mischung während einer Verweildauer von bis zu 4 h, besonders bevorzugt etwa 0,5 h bis 1,5 h, der Maximaltemperatur ausgesetzt wird.

21. Verfahren zur Herstellung eines Verdampferkörpers (21) nach einem der Ansprüche 8 bis 9, **dadurch gekennzeichnet, dass** wenigstens eine Faser und/oder wenigstens ein Filament umfassend den wenigstens einen thermoplastischen Kunststoff zu einem textilen Gebilde, vorzugsweise zu einem flächigen oder räumlichen Gewebe, verarbeitet wird bzw. werden, wobei aus dem textilen Gebilde der Verdampferkörper (21) oder ein Teil davon geformt wird.

22. Verfahren nach Anspruch 21, **dadurch gekennzeichnet, dass** ein Faden und/oder Garn umfassend die wenigstens eine Faser und/oder das wenigstens eine Filament zum textilen Gebilde verarbeitet wird bzw. werden.

23. Verfahren nach einem der Ansprüche 14 bis 22, **dadurch gekennzeichnet, dass** auf eine Oberfläche (3) des Verdampferkörpers (21) wenigstens bereichsweise wenigstens eine elektrisch leitfähige Beschichtung (4) aufgebracht wird.

24. Verfahren nach Anspruch 23, **dadurch gekennzeichnet, dass** die wenigstens eine elektrisch leitfähige Beschichtung (4) galvanisch auf die Oberfläche (3) des Verdampferkörpers (21) aufgebracht oder auf die Oberfläche (3) aufgedampft wird.

## Claims

1. An atomizer body (21) for an atomizer device (2) of an inhaler, in particular an electronic cigarette (1), wherein the atomizer body (21) can accommodate a liquid and consists of a material comprising at least one thermoplastic, **characterized in that** the material of the atomizer body (21) comprises at least one electrically conductive additive which is mixed with the at least one thermoplastic to provide a mixture.

2. The atomizer body according to claim 1, **characterized in that** a melting temperature of the at least one thermoplastic is greater than around 180°C, preferably greater than around 250°C, or a glass transition temperature of the at least one thermoplastic is greater than around 80°C, preferably greater than around 150°C.

3. The atomizer body according to any one of claims 1 to 2, **characterized in that** the atomizer body (21) consists substantially entirely of at least one polyaryletherketone, preferably polyetheretherketone.

4. The atomizer body according to any one of claims 1 to 3, **characterized in that** the atomizer body (21) has an open porosity of around 30% to 96%, preferably of around 50% to 95%, particularly preferably of around 65% to 94%.

5. The atomizer body according to any one of claims 1 to 4, **characterized in that** the material of the atomizer body (21) comprises at least one additive.

6. The atomizer body according to any one of claims 1 to 5, **characterized in that** the at least one electrically conductive additive is a metal, graphite, graphene, silicon carbide or activated carbon.

7. The atomizer body according to any one of claims 1 to 6, **characterized in that** one surface (3) of the atomizer body (21) is provided at least in places with at least one electrically conductive coating (4).

8. The atomizer body according to any one of claims 1 to 7, **characterized in that** the atomizer body (21) is configured at least in places, preferably substantially entirely, as a textile structure, preferably as a two-dimensional or three-dimensional woven fabric, wherein the textile structure comprises at least one fiber and/or at least one filament comprising the at least one thermoplastic.

9. The atomizer body according to claim 8, **characterized in that** the at least one fiber and/or the at least one filament consists or consist substantially entirely of at least one polyaryletherketone, preferably polyetheretherketone, and/or **in that** the at least one fiber and/or the at least one filament has or have a diameter of less than 1000 µm, preferably less than 500 µm, particularly preferably less than 300 µm.

10. An atomizer device (2) for an inhaler, in particular an electronic cigarette (1), having at least one atomizer body (21) according to any one of claims 1 to 9.

11. The atomizer device according to claim 10, **characterized in that** the atomizer device (2) comprises an accommodation space (22) for a liquid, wherein the atomizer body (21) is in connection with the liquid.

12. An inhaler, in particular electronic cigarette (1), having at least one atomizer device (2) according to any one of claims 10 to 11.

13. A mouthpiece (30) for an inhaler, in particular an electronic cigarette (1), having at least one atomizer device (2) according to any one of claims 10 to 11.

14. A method for producing an atomizer body (21) according to any one of claims 1 to 9, **characterized in that** a mixture comprising at least one thermoplastic is provided, wherein the mixture is heated until the mixture is at least superficially molten or a melt forms, wherein at least one atomizer body (21) is shaped from the at least superficially molten mixture or the melt, wherein the mixture comprises at least one electrically conductive additive which is mixed with the at least one thermoplastic to provide the mixture.

15. The method according to claim 13 or 14, **characterized in that** the mixture comprises at least one additive which is mixed with the at least one thermoplastic to provide the mixture.

16. The method according to claim 15, **characterized in that** the mixture comprises at least one soluble solid, preferably a salt, wherein the at least one soluble solid is mixed with the at least one thermoplastic to provide the mixture, wherein after shaping of the at least one atomizer body (21) the at least one soluble solid is dissolved at least in part, preferably substantially completely, out of the atomizer body (21).

17. The method according to any one of claims 13 to 16, **characterized in that** the at least one thermoplastic is a polyaryletherketone, preferably polyetheretherketone, in powder or flake form.

18. The method according to claim 17, **characterized in that** the mass fraction of the polyaryletherketone in powder or flake form in the mixture amounts to around 4 % to 65 %, preferably around 5 % to 55 %, particularly preferably around 6 % to 45 %.

19. The method according to claim 17 or 18, **characterized in that** polyetheretherketone in powder form is mixed with sodium chloride, wherein preferably the mass fraction of the polyetheretherketone in powder form in the mixture amounts to around 4% to 65%, particularly preferably around 5% to 55%, very particularly preferably around 6% to 45%, wherein preferably an average grain size of the polyetheretherketone in powder form is less than around 1000 µm, particularly preferably less than around 100 µm.

20. The method according to any one of claims 14 to 19, **characterized in that** the mixture is heated to a maximum temperature of between around 350°C and around 450°C, preferably to around 370°C, wherein preferably the mixture is exposed to the maximum temperature over a residence time of up to 4 h, particularly preferably around 0.5 h to 1.5 h.

21. A method for producing an atomizer body (21) according to any one of claims 8 to 9, **characterized in that** at least one fiber and/or at least one filament comprising the at least one thermoplastic is or are processed into a textile structure, preferably into a two-dimensional or three-dimensional woven fabric, wherein the atomizer body (21) or a part thereof is shaped from the textile structure.

22. The method according to claim 21, **characterized in that** a thread and/or yarn comprising the at least one fiber and/or the at least one filament is or are processed into the textile structure.

23. The method according to any one of claims 14 to 22, **characterized in that** at least in places at least one electrically conductive coating (4) is applied to a surface (3) of the atomizer body (21).

24. The method according to claim 23, **characterized in that** the at least one electrically conductive coating (4) is applied by electrodeposition to the surface (3) of the atomizer body (21) or vapor-deposited onto the surface (3).

## Revendications

1. Corps d'atomiseur (21) pour un dispositif d'atomiseur (2) d'un inhalateur, en particulier d'une cigarette électronique (1), le corps d'atomiseur (21) pouvant accueillir un liquide et se composant d'un matériau, lequel comprend au moins un plastique thermoplastique, **caractérisé en ce que** le matériau du corps d'atomiseur (21) comprend au moins un produit ajouté électriquement conducteur, lequel est mélangé avec l'au moins un plastique thermoplastique pour la mise à disposition d'un mélange.

2. Corps d'atomiseur selon la revendication 1, **caractérisé en ce qu'**une température de fusion de l'au moins un plastique thermoplastique est supérieure à environ 180 °C, de préférence supérieure à environ 250 °C, ou **en ce qu'**une température de transition vitreuse de l'au moins un plastique thermoplastique est supérieure à environ 80 °C, de préférence supérieure à environ 150 °C.

3. Corps d'atomiseur selon l'une des revendications 1 à 2, **caractérisé en ce que** le corps d'atomiseur (21) se compose essentiellement d'au moins un polyaryléthercétone, de préférence un polyétheréthercétone.

4. Corps d'atomiseur selon l'une des revendications 1 à 3, **caractérisé en ce que** le corps d'atomiseur (21) présente une porosité ouverte d'environ 30 % à 96 %, de préférence d'environ 50 % à 95 %, en particulier de préférence d'environ 65 % à 94 %.

5. Corps d'atomiseur selon l'une des revendications 1 à 4, **caractérisé en ce que** le matériau du corps d'atomiseur (21) comprend au moins un additif.

6. Corps d'atomiseur selon l'une des revendications 1 à 5, **caractérisé en ce que** l'au moins un produit ajouté électriquement conducteur est un métal, un graphite, un graphène, un carbure de silicium ou un charbon actif.

7. Corps d'atomiseur selon l'une des revendications 1 à 6, **caractérisé en ce qu'**une surface (3) du corps d'atomiseur (21) est munie d'au moins un revêtement électriquement conducteur (4), au moins par zones.

8. Corps d'atomiseur selon l'une des revendications 1 à 7, **caractérisé en ce que** le corps d'atomiseur (21) est constitué au moins par zones, de préférence essentiellement totalement, comme une structure textile, de préférence comme un tissu plan ou tridimensionnel, la structure textile comprenant au moins une fibre et/ou au moins un filament comprenant l'au moins un plastique thermoplastique.

9. Corps d'atomiseur selon la revendication 8, **caractérisé en ce que** l'au moins une fibre et/ou l'au moins un filament se compose, ou se composent, entièrement d'au moins un polyaryléthercétone, de préférence de polyétheréthercétone, et/ou **en ce que** l'au moins une fibre et/ou l'au moins un filament présente, ou présentent, un diamètre inférieur à 1 000 µm, de préférence inférieur à 500 µm, en particulier de préférence inférieur à 300 µm.

10. Dispositif d'atomiseur (2) pour un inhalateur, en particulier une cigarette électronique (1), avec au moins un corps d'atomiseur (21) selon l'une des revendications 1 à 9.

11. Dispositif d'atomiseur selon la revendication 10, **caractérisé en ce que** le dispositif d'atomiseur (2) comprend un espace d'accueil (22) pour un liquide, le corps d'atomiseur (21) étant en contact avec le liquide.

12. Inhalateur, en particulier cigarette électronique (1), avec au moins un dispositif d'atomiseur (2) selon l'une des revendications 10 à 11.

13. Embout (30) pour un inhalateur, en particulier cigarette électronique (1), avec au moins un dispositif d'atomiseur (2) selon l'une des revendications 10 à 11.

14. Procédé pour la réalisation d'un corps d'atomiseur (21) selon l'une des revendications 1 à 9, **caractérisé en ce qu'**un mélange comprenant au moins un plastique thermoplastique est mis à disposition, le mélange étant chauffé jusqu'à ce que le mélange soit fondu au moins superficiellement ou jusqu'à ce qu'une fusion se forme, au moins un corps d'atomiseur (21) étant formé par l'au moins un mélange fondu superficiellement ou par la fusion, le mélange comprenant au moins un produit ajouté électriquement conducteur, lequel est mélangé avec l'au moins un plastique thermoplastique pour la mise à disposition du mélange.

15. Procédé selon la revendication 13 ou 14, **caractérisé en ce que** le mélange comprend au moins un additif, lequel est mélangé avec l'au moins un plastique thermoplastique pour la mise à disposition du mélange.

16. Procédé selon la revendication 15, **caractérisé en ce que** le mélange comprend au moins un solide soluble, de préférence un sel, l'au moins un solide soluble étant mélangé avec l'au moins un plastique thermoplastique pour la mise à disposition du mélange, l'au moins un solide soluble étant extrait au moins partiellement, de préférence totalement, du corps d'atomiseur (21), après la formation de l'au moins un corps d'atomiseur (21).

17. Procédé selon l'une des revendications 13 à 16, **caractérisé en ce que** l'au moins un plastique thermoplastique est un polyaryléthercétone, de préférence un polyétheréthercétone, en poudre ou en flocons.

18. Procédé selon la revendication 17, **caractérisé en ce que** la fraction massique dans le mélange du polyaryléthercétone en poudre ou en flocons s'élève à d'environ 4 % à 65 %, de préférence d'environ 5 % à 55 %, en particulier de préférence d'environ 6 % à 45 %.

19. Procédé selon la revendication 17 ou 18, **caractérisé en ce que** le polyétheréthercétone en poudre est mélangé avec du chlorure de sodium, la fraction massique dans le mélange du polyétheréthercétone en poudre s'élevant de préférence d'environ 4 % à 65 %, en particulier de préférence d'environ 5 % à 55 %, tout particulièrement de préférence d'environ 6 % à 45 %, une taille de grain moyenne du polyétheréthercétone en poudre étant de préférence inférieure à 1 000 µm, en particulier de préférence inférieure à 100 µm.

20. Procédé selon l'une des revendications 14 à 19, **caractérisé en ce que** le mélange est chauffé à une température maximale entre environ 350 °C et environ 450 °C, de préférence à environ 370 °C, le mélange étant de préférence exposé à la température maximale pendant une durée atteignant 4 h, en particulier de préférence d'environ 0,5 h à 1,5 h.

21. Procédé pour la réalisation d'un corps d'atomiseur (21) selon l'une des revendications 8 à 9, **caractérisé en ce que** l'au moins une fibre et/ou l'au moins un filament comprenant l'au moins un plastique thermoplastique est transformé, ou sont transformés, en une structure textile, de préférence en un tissu plan ou tridimensionnel, le corps d'atomiseur (21) ou une partie de celui-ci étant formé à partir de la structure textile.

22. Procédé selon la revendication 21, **caractérisé en ce qu'**une ficelle et/ou un fil comprenant l'au moins une fibre et/ou l'au moins un filament est transformé, ou sont transformés, en ladite structure textile.

23. Procédé selon l'une des revendications 14 à 22, **caractérisé en ce qu'**au moins un revêtement électriquement conducteur (4) est appliqué sur une surface (3) du corps d'atomiseur (21), au moins par zones.

24. Procédé selon la revendication 23, **caractérisé en ce que** l'au moins un revêtement électriquement conducteur (4) est appliqué galvaniquement sur la surface (3) du corps d'atomiseur (21) ou est vaporisé sur la surface (3).
